(19) Europäisches Patentamt / European Patent Office / Office européen des brevets

(11) **EP 4 166 076 A1**

(12) **EUROPEAN PATENT APPLICATION**

(43) Date of publication:
**19.04.2023 Bulletin 2023/16**

(21) Application number: **21306449.6**

(22) Date of filing: **15.10.2021**

(51) International Patent Classification (IPC):
**A61B 5/11** *(2006.01)*  **A61B 5/00** *(2006.01)*

(52) Cooperative Patent Classification (CPC):
**A61B 5/112; A61B 5/1123; A61B 5/6801**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Université de Rennes 1**
  **35065 Rennes Cedex (FR)**

• **Institut National de la Santé et de la Recherche Médicale (INSERM)**
  **75013 Paris (FR)**

(72) Inventors:
• **ABBAS, Manuel**
  **35700 RENNES (FR)**
• **LE BOUQUIN JEANNES, Régine**
  **35760 SAINT-GRÉGOIRE (FR)**

(74) Representative: **Lavoix**
  **2, place d'Estienne d'Orves**
  **75441 Paris Cedex 09 (FR)**

(54) **METHOD AND DEVICE FOR PREDICTING FRAILTY OF A SUBJECT**

(57)   This method for predicting frailty of a subject, using a wearable device, comprising an accelerometer, worn in unsupervised conditions, comprising:
• computing (58) a plurality of global features representative of the acceleration signals captured during a predetermined observation period,
• extracting (60) segments of acceleration signals within at least one period of walking of the subject within the observation period and computing (62) a plurality of local features representative of the subject's gait,
• applying a first frailty assessment module (64) on the plurality of global features to obtain a first output representative of the frailty of the subject,
• applying a second frailty assessment module (66) on the plurality of local features to obtain a second output representative of the frailty of the subject,
• combining (68) the first and second outputs (64, 66) to predict a status of frailty of the subject amongst the classes {robust, pre-frail, frail} for the predetermined observation period.

EP 4 166 076 A1

FIG.2

**Description**

The present invention concerns a method and device for predicting frailty of a subject.

The invention belongs to the domain of healthcare monitoring systems, and more particularly the monitoring of frailty in elderly persons.

The frailty of elderly persons is becoming increasingly important, since the frailty syndrome is representative of decline in health and physical functions, and increases the vulnerability of the person. Such a frailty syndrome has a prevalence in older persons, and given the generalized aging of the population in industrialized countries, detecting and predicting the frailty of a subject becomes a priority.

In this context, frailty has been a research topic in the past two decades. The frailty of a subject may be classified into three classes which are: robust (full physical performance), pre-frail (clinically silent), frail (progressive clinical worsening), the class "frail" being previous to the lack of autonomy of a subject, the subject being then considered disabled.

In the article "Frailty in older adults: Evidence for a phenotype" by L.P. Fried et al, published in the Journal of Gerontology: Medical sciences 56A, 2001, pages 146-156, a phenotype of frailty includes criteria such as unintentional weight loss, exhaustion, low physical activity, slow walking speed and weakness. In particular, the gait speed, which is the ratio of walked distance to the time spent, was considered to be a relevant indicator of frailty.

It is known to use a wearable device, which is worn by the subject, for example a connected watch, to monitor some physical activities, including walking.

Most works on frailty assessment include a supervised phase in which the subject is asked to walk a given distance while wearing a wearable device. However, this approach has its limits, since such a supervised frailty assessment can only be carried out once in a while, in a clinical environment, to evaluate the health condition of the subject.

Patent US 10,299,736 B2 describes a method, device and system for diagnosing and monitoring frailty using a wearable sensor module, configured to be attached to a person, the sensor comprising an accelerometer component configured to generate signals in response to motion or movements of the body, from which frailty related biomarkers are extracted. Such a sensor module is worn in unsupervised conditions. Furthermore, a physiological sensor is used, and the method proposed uses also physiological responses such as heart rate, respiration rate or skin temperature. Therefore, the method proposed in this patent necessitates a plurality of worn sensors to acquire several types of physiological data.

There is a need to provide a method and system providing simpler and more accurate parameters to predict a frailty status of a subject, and further to detect the changeover from robust to pre-frail, or from pre-frail to frail, so as to better take into account the frailty of the subject.

This and other objects are achieved by a method for predicting frailty of a subject, using a wearable device, worn by the subject in unsupervised conditions, the wearable device comprising an accelerometer, the method being implemented by a microcontroller, connected to the accelerometer, the microcontroller being configured to receive acceleration signals from the accelerometer over time. The method being comprises the following steps, implemented by the microcontroller:

(a) compute a plurality of global features representative of the acceleration signals captured during a predetermined observation period,
(b) extract segments of acceleration signals within at least one period of walking of the subject within the observation period;
(c) compute a plurality of local features representative of the subject's gait during said at least one period of walking from the magnitude of the segments of acceleration signals,
(d) apply a first frailty assessment module on the plurality of global features to obtain a first output representative of the frailty of the subject,
(e) apply a second frailty assessment module on the plurality of local features to obtain a second output representative of the frailty of the subject,
(f) combine the first output of the first frailty assessment module and the second output of the second frailty assessment module to predict a status of frailty of the subject amongst the classes {robust, pre-frail, frail} for the predetermined observation period.

Advantageously, the method achieves frailty prediction using global features and local features obtained from processed segments of acceleration signals.

In embodiments of the invention, the method for predicting frailty of a subject, considered alone or according to all technically possible combinations.

The first output is a first modified vector obtained from a first feature vector of global features and the second output is a second modified vector obtained from a second feature vector of local features, the combining comprising concatenating said first modified vector and said second modifier vector into a concatenated vector, the combining comprising providing the concatenated vector to an output layer to obtain the predicted status of frailty of the subject.

The first output is a first estimated status of frailty of the subject, the second output is a second estimated status of frailty of the subject, and the combining comprises voting to select the predicted status of frailty of the subject between the first estimated status of frailty of the subject and the second estimated status of frailty of the subject.

The method comprises applying the steps (a) to (f) over a plurality of successive observation periods to obtain a predicted status of frailty of the subject associated to each observation period of the plurality of successive observation periods, the method further comprising an analysis step obtaining a trajectory representative of the frailty of the subject over the plurality of successive observation periods, and determining, by analysing said trajectory, a transition between robust to pre-frail or between pre-frail to frail.

The method comprises a processing of the acceleration signals during the observation period to obtain a list of labels representing a sequence of daily physical activities performed by the subject wearing the wearable device, and further comprises determining active periods and inactive periods based on said list of labels.

The method further comprises computing a plurality of global features relative to the determined active periods during the observation period.

The method further comprises acquiring barometric signals during the observation period and computing at least one altitude related global feature.

The method further comprises computing at least one sleep quality related global feature by analysing acceleration signals captured during a predetermined night period.

The computing of a plurality of local features comprises computing a normalized auto-correlation signal of the magnitude of the segments of acceleration signals during at least one period of walking, and computing at least one local feature based on said normalized auto-correlation signal.

The computing of a plurality of local features further comprises fitting an autoregressive model to the magnitude of the segments of acceleration signals during at least one period of walking, the fitting comprising computing parameters of the autoregressive model, and computing a plurality of local features based on the parameters of the autoregressive model.

The first frailty assessment module applies a machine learning classifier on the computed global features.

The second frailty assessment module applies a machine learning classifier on the computed local features.

The invention relates also to a device for predicting frailty of a subject, the device being a wearable device, worn by the subject in unsupervised conditions, comprising an accelerometer and a microcontroller connected to the accelerometer, the microcontroller being configured to receive acceleration signals from the accelerometer over time. The microcontroller is configured to implement:

- an extracting module configured to extract segments of acceleration signals within at least one period of walking of the subject within an observation period,
- a global features computing module configured to compute a plurality of global features representative of the acceleration signals captured during a predetermined observation period,
- a local features computing module configured to compute a plurality of local features representative of the subject's gait during said at least one period of walking from the magnitude of the segments of acceleration signals,
- a first frailty assessment module applied on the plurality of global features to obtain a first output representative of the frailty of the subject,
- a second frailty assessment module applied on the plurality of local features to obtain a second output representative of the frailty of the subject,
- a fusion module configured to combine the first output of the first frailty assessment module and the second output of the second frailty assessment module to predict a status of frailty of the subject amongst the classes {robust, pre-frail, frail} for the predetermined observation period.

The device for predicting frailty of a subject is configured for implementing all the features of the method for predicting frailty of a subject as described above.

The invention relates also to a computer program comprising code instructions which, when they are executed by a programmable device, implement a method for predicting frailty of a subject as briefly described above.

Further characteristics and advantages of the present invention will become apparent from the following description, provided merely by way of non-limiting examples, with reference to the enclosed drawings, in which:

- Figure 1 shows a system comprising a device for predicting frailty of a subject according to an embodiment of the present invention;
- Figure 2 is a flowchart of the main steps of a method for predicting frailty of a subject according to an embodiment;
- Figure 3 is a flowchart of the global features computation according to an embodiment;
- Figure 4 is a flowchart of the local features computation according to an embodiment.

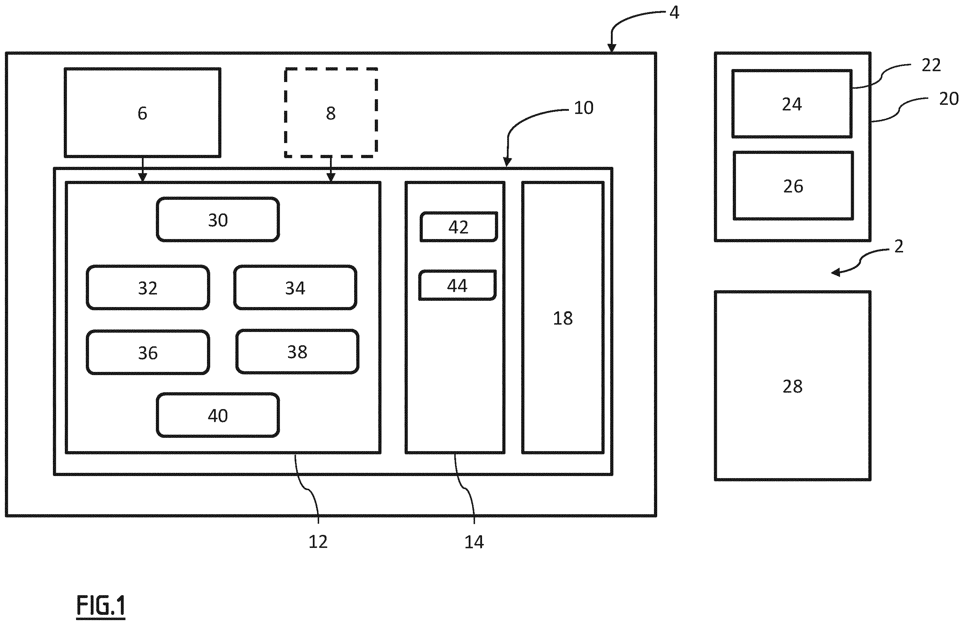

**Figure 1** illustrates a system 2 for predicting frailty of a person according to an embodiment of the invention.

The monitoring system 2 comprises a wearable device 4, which can be worn autonomously by a person in everyday life.

For example, the wearable device 4 is a smart belt positioned around the waist, or the chest or the hip of a user. Advantageously, such a wearable device is positioned close to the center of body mass of the person. In the following, a person wearing such a device is called a subject.

According to an alternative, the wearable device is worn around the neck, for example in the form of a necklace.

According to an embodiment, the wearable device 4 is a smart belt, comprising a built-in accelerometer 6 adapted to acquire acceleration data.

Preferably, the accelerometer 6 is a tri-axial accelerometer, providing three acceleration components according to three mutually orthogonal axes of a 3D referential. Such an accelerometer provides three acceleration components, each comprising data representative of the linear acceleration and the gravity acceleration according to one direction, referenced hereafter as $A_x$, $A_y$, $A_z$. The accelerometer is a sensor configured to acquire data over time, the outputs of the accelerometer being samples of three raw acceleration signals over time.

According to an embodiment, the accelerometer 6 uses MEMS (for Micro-Electro Mechanical Systems) technology, and has a sampling frequency up to 100 Hz or 1 KHz, for example equal to 50Hz.

Optionally, the wearable device 4 further comprises a barometer 8, adapted to acquire pressure data.

The wearable device 4 further comprises a microcontroller 10, which is a programmable electronic device, adapted to receive the raw data acquired by the accelerometer 6, for example via a wired connection, for example on a Printed Circuit Board.

The microcontroller 10 comprises a processor 12, one or more electronic memory units 14 and an interface unit 18, which are all connected via internal communication buses.

The interface unit 18 is adapted to communicate data, using radio communication technology for example, to an external device 20, which comprises a human-machine interface 22.

The external device 20 is for example a smartphone, a pad, a laptop etc. The human-machine interface 22 comprises a display screen 24, and a command module 26 adapted to receive commands from a user. According to an embodiment, the display 24 is a tactile display, which serves for both displaying and acquiring user commands.

According to an alternative embodiment, the wearable device 4 also comprises a human-machine interface, for example comprising a display screen (not shown).

Furthermore, according to an embodiment, the interface unit 18 is also adapted to communicate data to a distant server 28. For example, server 28 is a server of medical data, the data being provided to medical staff for health monitoring of a subject.

The system 2 for monitoring physical activities of a subject is configured to acquire acceleration signals from the accelerometer 6 and to process these signals according to a method for predicting frailty of a subject which will be described in detail hereafter.

The microcontroller 10 is configured to implement several processing modules including :

- an extraction module 30 configured to extract segments of acceleration signals within at least one period of walking of the subject in unsupervised conditions;

- a global features computing module 32 configured to compute a plurality of global features representative the acceleration signals captured during a predetermined observation period;

- a local features computing module 34 configured to compute a plurality of local features representative of the subject's gait during said at least one walking period from the magnitude of extracted segments of acceleration signals;

- a first frailty assessment module 36 configured to process the plurality of global features to obtain a first output representative of the frailty of the subject;

- a second frailty assessment module 38 configured to process the plurality of local features to obtain a second output representative of the frailty of the subject;

- a fusion module 40 configured to combine the first and the second outputs to predict the status of frailty of the subject, as described in detail hereafter.

For example, the observation period is a 24 hours period, and the periods of walking are of variable duration depending on physical activities of the subject during each observation period.

The so-called global features are features computed using the acceleration signals captured during an observation period, the observation period having a given duration, e.g. 24 hours, the observation period including one or several periods of walking.

The so-called local features are features representative of the subject's gait, computed from at least one period of walking, using the magnitude of extracted segments of acceleration signals.

The subject uses the wearable device in unsupervised conditions, i.e. during daily life activities and for an undetermined period of time, as opposed to supervised conditions, in which a subject wears such a wearable device during an test or exercise, for example in a clinical environment, for a given short period of time.

According to an embodiment, modules 30, 32, 34, 36, 38 and 40 are implemented as software, in the form of a computer program, stored in an electronic memory 14 of the system 2 for monitoring physical activities, and executed by the processor 12.

Alternatively, the modules 30, 32, 34, 36, 38 and 40 are stored on a non-volatile information recording medium, such as an optical disk, a magneto-optical disk, any type of non-volatile memory (e.g. EPROM, EEPROM, FLASH, NVRAM), a magnetic card or and optical card.

In an alternative embodiment, each of the modules 30, 32, 34, 36, 38 and 40 is implemented by a FPGA (Field Programmable Gate Array), or a dedicated integrated circuit such as an ASIC (Applications Specific Integrated Circuit).

According to an embodiment, the acceleration signals acquired by the accelerometer are segmented using a sliding window, and the time series of windowed segments of acceleration signals 42 are stored in the memory 14. A window designates a time period, having a starting time, an ending time and a duration.

The result of the processing by the modules 30, 32, 34, 36, 38 and 40 is a frailty status of the subject 44 which is also stored in the memory 14.

The predicted frailty status may be computed repetitively over each observation period over a plurality of successive observation periods, e.g. each day over one or two month(s), and stored, forming a frailty status signal, also called trajectory, evolving over time, which may further be analyzed to accurately predict the frailty status of the subject.

Each of the modules 36, 38 and 40 implements a machine learning process.

The modules 36, 38 and 40 are trained on a database of registered data over observation periods on three distinct populations of subjects, each population comprising subjects already categorized in one of the categories « robust », « pre-frail » and « frail ».

As is known in the field of machine learning, hyperparameters are used to define a machine learning model or classifier, for example the number of neurons and the activation function of a neural network. The hyperparameters are used to control the learning process.

For each module 36, 38, 40, the hyperparameters as well as the weight values obtained during the training process are stored in the memory unit 14, and are further used to predict the frailty status of a subject when the monitoring begins.

According to one embodiment, each frailty status class is labelled with, for example, a number, the robust class is labelled "3", the pre-frail class is labelled 2 and the frail class is labelled 1. The trajectory is then formed by a series of values.

According to an embodiment, when a transition from a frailty class to another is predicted or detected, an alarm is sent, for example to medical staff and/or to family members so as to warn them of the physical weakening of the subject.

Furthermore, the analysis of the predicted frailty status over time helps stabilizing the response of the system in case of misclassification over one observation period, which may appear as an outlier compared to the plotted trajectory.

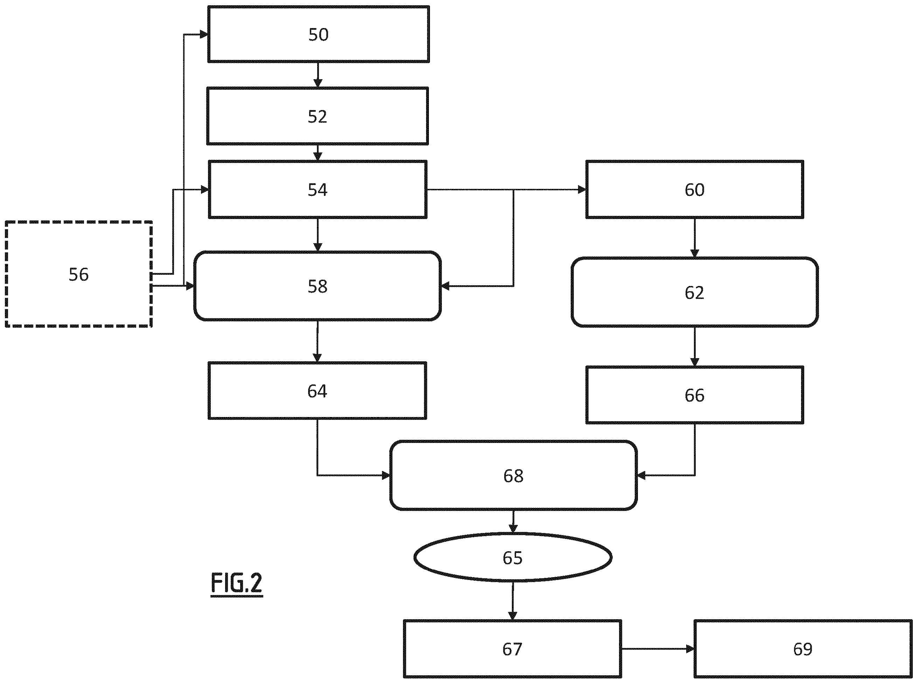

**Figure 2** is a flowchart of the main steps of a method for predicting frailty of a subject according to an embodiment, to be implemented by a processor of a programmable as described above.

The method comprises a step 50 of obtaining the acceleration signals as provided by the accelerometer which is included in the wearable device, during an observation period.

The observation period is a predetermined period of time, such as for example 24 hours or less. As exposed above, the predicted frailty status is computed on several successive observation periods forming a longer monitoring period (several days, weeks or months).

The acceleration signals are time varying signals (time series), for example provided by a 3D accelerometer and comprising a three time varying signal ax(t), av(t), az(t) corresponding to three spatial axis of an orthogonal spatial referential. The acceleration signals are acquired with a given sampling frequency, typically comprised between 25Hz to 50 Hz, and preferably higher than 40Hz.

The acceleration signals are then processed in processing step 52 into time series of segments of acceleration signals. Preferably, each acceleration signal ax(t), av(t), az(t) is decomposed into overlapping segments.

Optionally, barometric signals are also obtained during the observation period (step 56).

The time series of segments of acceleration signals are processed in processing step 54 to determine active periods vs inactive periods.

Preferably, the time series of segments of acceleration signals are processed using a method as described in the article "Exploiting Local Temporal Characteristics via Multinomial Decomposition Algorithm for Real-Time Activity Recognition," by M. Abbas and R. Le Bouquin Jeannes, published in IEEE Transactions on Instrumentation and Measurement, vol. 70, September 2021, for obtaining labels representing a sequence of physical activities carried out during the observation period, and an associated time period for each physical activity.

The labels correspond to the following categories of physical activities: 'sitting-down', 'standing-up', 'lying-down', 'rising up', 'walking, jogging', 'staying still' and 'other'. The category 'other' indicates that the processing did not classify a given segment of acceleration signals into any of the labelled categories.

Alternatively, other methods known in the prior art may be applied to the acceleration signals received during the observation period to discriminate between walking periods, other active periods and inactive periods. For example, the method applied is the method described in the article "Weightlessness feature — a novel feature for single tri-axial accelerometer based activity recognition," by Zhenyu He, Zhibin Liu, Lianwen Jin, Li-Xin Zhen and Jian-Cheng Huang, published in 2008 19th International Conference on Pattern Recognition, 2008.

Furthermore, if barometric signals are also obtained in a barometric signals obtaining step 56, the method further distinguishes between 'walking', 'going upstairs' and 'going downstairs', the activities 'going upstairs' and 'going downstairs' corresponding to sub-categories of walking with changes in altitude.

The method further comprises a step 58 of computing global features which are characteristic of the subject's physical activity during the observation period, using the acceleration signals captured.

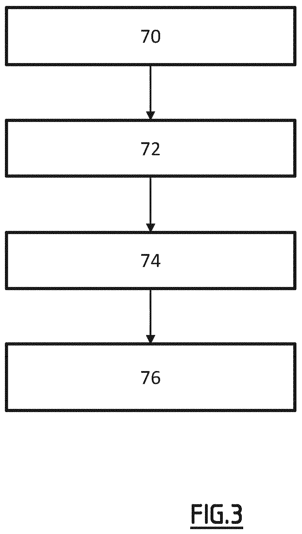

An example of computing global features will be detailed hereafter with respect to figure 3.

Further, the method comprises more specifically a step 60 of extracting segments of acceleration signals of periods labelled as walking periods (also referred to as "periods of walking'), and the computation 62 of local features representative of the subject's gait.

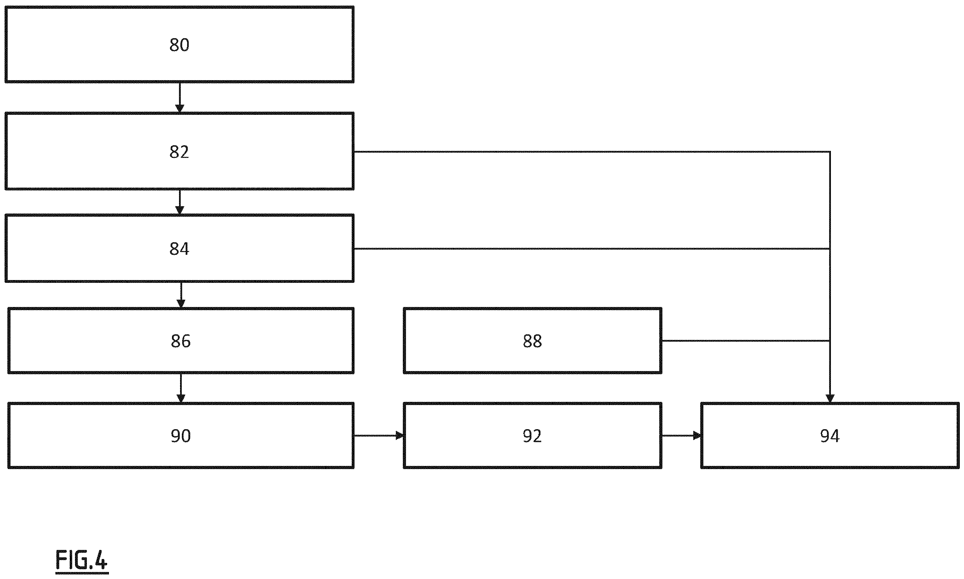

An example of computing local features will be detailed hereafter with respect to figure 4.

The computed global features are stored, preferably in an array or first feature vector denoted by $G$, and transmitted to a first frailty assessment module 64.

According to a first embodiment, the first frailty assessment module 36 applies for example a thresholding process or a machine learning trained process to compute a first estimated status of frailty amongst robust, pre-frail, frail, and the first estimated status of frailty is forwarded to the combination step 68.

According to a second embodiment, the first frailty assessment module 36 consists of fully connected layers (dense layers) which takes the first feature vector G as an input and models the first feature vector G into a first modified vector $\tilde{G}$ that is forwarded to the combination step 68.

The computed local features are stored, preferably in an array or second feature vector denoted by L transmitted to a second frailty assessment module 66 which applies for example a machine learning trained process to compute a second output.

According to a first embodiment, the second output is a second estimated status of frailty, and the machine learning process comprises: Random Forests, Gradient Boosting Machine, Neural networks, or another classifier. Implementations of such machine learning processes are known.

According to a second embodiment, the second frailty assessment module 38 consists of fully connected layers (dense layers) which takes the second feature vector L as an input and models it into a second modified vector $\tilde{L}$ that is forwarded to the combination step 68.

Finally, the first and the second outputs of respectively the frailty assessment modules 36, 38 are combined during a combination step 68, to obtain a frailty status 65 of the subject.

According to one embodiment, the first and the second frailty assessment modules 36 and 38 are composed with the same architecture of connected layers or with the same machine learning classifier. For example, each of the modules 36, 38 implements an SVM (support vector machine).

According to another embodiment, the first and the second frailty assessment modules 36 and 38 are composed with a different architecture of connected layers or with different machine learning classifiers.

According to the first embodiment, the fusion module 40 receives the first estimated status of frailty and the second estimated status of frailty and applies a voting method to determine a predicted frailty status. If each of the first frailty assessment module and the second frailty assessment module provide the same predicted status of frailty, then said predicted status of frailty is chosen as the result of the combination by majority voting.

If there is a discrepancy between the first output and the second output, the combining step applies a voting based on a rule. For example, in a highly sensitive system, the worst prediction is kept.

According to the second embodiment, when the first output is a first modified vector $\tilde{G}$ of first dimension N1 and the second output is a second modifier vector $\tilde{L}$ of second dimension N2, the combination step 68 combines both outputs by concatenating the first vector and the second vector to obtain a concatenated vector $\tilde{F}$ of dimension N1+N2.

The concatenated vector $\tilde{F}$ is input to an output layer, such as for example a softmax layer, which generates a probability distribution over 3 classes and furthermore the probability of occurrence of each of the classes, to predict the output, i.e. the frailty status.

Furthermore, the predicted frailty status for each observation period is stored at storing step 67 over a plurality of successive observation periods, and an analysis step 69 is applied. For example, a frailty trajectory is computed and analysis of the trajectory is applied to determine or to predict the transition over time from robust to pre-frail or from pre-frail to frail. Optionally, when a transition towards more frailty is predicted, an alarm is raised.

**Figure 3** is a flowchart of the main steps of the computation of global features, according to an embodiment.

The computation of global features comprises a first step 70 of determining active and inactive periods.

According to an embodiment, the acceleration signals and the barometric signals are processed to obtain labels representing a sequence of physical activities carried out during the observation period, and associated time periods for each physical activity, the activities labelled as 'standing-up', 'rising up'; 'walking', and, optionally, 'going upstairs' and 'going downstairs'. The physical activities 'sitting-down', and 'lying-down' and 'jogging' are categorized as active, while 'staying still' is categorized as inactive.

Several global features are computed during the step 72 of computing activity related global features.

From the respective durations of active periods and inactive periods, a first global feature computed is the activity rate G1, i.e. the percentage of duration of active periods during the observation period.

The periodicity of movements is the second global feature G2 computed. When the subject is moving, the autocorrelation of the corresponding acceleration data is computed. The periodicity cycles constitute this second global feature.

Furthermore, the number of steps G3 during the observation period is computed as a global feature by identifying the peaks in the acceleration signals when the subject is standing and moving.

The global features further include a fourth global feature G4 which is representative of the energy expenditure, computed as the MET (for Metabolic Equivalent) resulting from the relationship between the intensity of the physical activity and the energy expenditure:

$$MET = \frac{BurnedCalories_{(kcal)}}{Duration_{(h)} \times Weight_{(kg)}}$$

The quantity of burned calories is estimated based on the intensity of physical activities, estimated from the magnitude of the acceleration signals during the active periods, for example by using a predetermined chart. The MET values are determined using a predefined table that links these values to the intensity of the movement, for example according to the teachings of the article "Physical activity and public health: updated recommendation for adults from the American College of Sports Medicine and the American Heart Association » by W. Haskell et al, published in Medicine & Science in Sports & Exercise. 2007;39(8):1423-1434

When using the barometric signals, the method further comprises a step 74 of computing an altitude related global feature.

This computation 74 comprises computing time series of altitude signals using barometric signals, the temperature and atmospheric pressure.

An altitude related global feature G5 is then computed from time series representing the altitude of the wearable device, for example a feature relative to the slope of the altitude signals. According to an embodiment, a barometric curve is computed, and the slope of the barometric curve and the difference in altitude indicate whether the subject is going upstairs or downstairs. For example, the global feature G5 indicates the number of times the subject takes the stairs (upstairs or downstairs) during the observation period.

Finally, the method comprises a step 76 of computing at least one global feature G6 evaluating the sleep of the subject. For example, the segments of acceleration signals corresponding to a night period, for example a period between 10 pm and 8 am, when the subject is lying down, are considered. Then sleeping cycles which last at least a minimal period of time, e.g. 5 minutes, are detected. A sleeping cycle is a period during which the subject is keeping the same position while sleeping, more precisely when the subject is lying down during the night period, without any rotation of interruption in the sleep pattern. The sleep pattern is determined from the shape of the acceleration signals.

The global feature G6 is for example computed as the average duration of the sleeping cycles, or as the overall duration of the sleeping cycles divided by the duration when the subject lying down during the night period.

riod.

In the embodiment described above, 6 global features G1 to G6 are computed, respectively 4 activity related features, 1 altitude related feature and 1 sleep quality related feature.

According to alternatives, only a subset of these 6 global features may be computed or several other features, for example several features relative to the sleep quality may be computed and further fed to the first frailty assessment module.

The set of subset of global features computed is stored, preferably in a first feature vector G as described above.

**Figure** 4 is a flowchart of the main steps of the computation of local features, according to an embodiment.

As explained above, the local features are features that relate to the gait of the subject.

The local features are computed from segments of acceleration signals during the periods of walking.

The method comprises a step 80 of computing the magnitude of the acceleration signals captured by the accelerometer.

The magnitude is computed for each sampling instant t as:

$$\|a(t)\| = \sqrt{a_X^2(t) + a_Y^2(t) + a_Z^2(t)}$$

It was noted that the range of acceleration magnitude, representative of the intensity of the gait, decreases when the subject becomes frail.

A first local feature F1 representative of the subjects gait is computed (step 82) as the K percent trimmed range of acceleration magnitude, with K=25% for example. The first local feature is computed for each segment of acceleration magnitude, each segment having a given first duration, for example 6 seconds.

The trimmed range of acceleration magnitude is computed as the range of the acceleration magnitude after excluding the k highest and lowest values, where:

$$k = \frac{N}{2} \times \frac{K}{100}$$

Where K is the percent and N is the length (i.e. the number of samples) of a segment. For example, when the segment duration is 6 seconds and the sampling frequency is 25Hz, N=6x25 samples.

Furthermore, the method comprises a computation step 84 of a second local feature F2 which is equal to the steps rate or cadence over a given second duration, preferably equal to the first duration (or segment duration), i.e. 6 seconds in the example given above. The steps rate is defined as the number of steps per said fixed duration.

The walking steps are considered to correspond to peaks in the acceleration magnitude signal. Any of the known methods to detect the peaks of a signal may be used.

Furthermore, the method comprises computing local features representative of the periodicity of the acceleration signals of walking periods.

To that end, the method comprises a step 86 of computing normalized autocorrelation of the acceleration magnitude signals of each walking period.

Advantageously, the autocorrelation signal provides information related to the periodicity of the gait movements.

The auto-correlation is denoted $R(\tau)$, the variable $\tau$ being also called a lag.

According to an embodiment, the auto-correlation is computed using a Fourier transform according to the following substeps. The acceleration magnitude signal is centered, in other words the mean value of the signal is subtracted from each sample, to obtain a signal denoted $\|\tilde{a}\|$ Then a Fourier transform, for example a FFT (Fast Fourrier Transform) is applied:

$$F_R(f) = FFT(\|\tilde{a}(t)\|)$$

Next, the result of the Fourier transform is multiplied by its complex conjugate:

$$M(f) = F_R(f) \times F_R^*(f)$$

Where $F_R^*$ designates the complex conjugate of $F_R$

Finally, the inverse fast Fourier transform, denoted IFFT, is applied to compute the auto-correlation in the time domain:

$$R(\tau) = IFFT(M(f))$$

The auto-correlation is normalized, i.e. each sample is divided by R(0) which is the maximal value.

Another local feature F3 is then computed at computation step 88 as a statistical moment of the normalized auto-correlation signal, preferably the skewness is representative of the asymmetry of the auto-correlation.

Alternatively, the local feature F3 is a different parameter computed from the autocorrelation, for example the standard deviation or the entropy of the auto-correlation signal.

The local feature F3 is representative of the periodicity of the magnitude of the acceleration during the considered period of walking.

Furthermore, it is proposed to fit an autoregressive (AR) model of given order to the time series of the acceleration magnitude signal during a walking period.

An auto-regressive model of time series signal $X_t$ is given by the formula:

$$X_t = b + \sum_{i=1}^{p} \phi_i X_{t-1} + \varepsilon_t$$

Where b is the bias which is a constant value, p is the order of the model, typically an integer between 1 and 8, $\varepsilon_t$ is a white noise and $\phi_i$ are the parameters of the model.

The parameters of an autoregressive model for modelling the acceleration magnitude $\|a(t)\|$ are determined at determination step 90.

According to an embodiment, an optimal order p is computed using the Akaike Information Criterion (AIC) or the Bayesian Information Criterion (BIC).

Any other known method for computing an optimal order p may be applied.

Next, local features are computed (step 92) from the autoregressive model, preferably by computing one or several statistical features of the coefficients of the model.

According to an embodiment, the local feature F4 computed is the distribution entropy of $\phi_i$, quantifying the unpredictability or the randomness of this set of parameters. Alternatively, the local feature F4 is computed from the standard deviation of the parameters $\phi_i$.

The local feature F5 is the kurtosis of $\Phi_i$, indicating whether the distribution of the set of parameters is tight or flattened. Alternatively, a different statistical moment of the distribution of the set of parameters may be used.

Finally, all the computed local feature F1 to F5 are scaled (step 94) using the sigmoidal transformation:

$$\hat{X} = \frac{1}{1 + \exp(-\dfrac{X - \mu_X}{\sigma_X})}$$

Where $\hat{X}$ is the scaled feature vector, X is the original feature vector, $\mu_x$ is the mean value of X and $\sigma_x$ is the standard deviation of X.

All scaled feature values are comprised in a predetermined interval, which is the interval [0,1] when applying the sigmoidal transformation.

The scaled vector of local features forms the second feature vector L mentioned above, which is provided as an entry to the second frailty assessment module.

In a preferred embodiment, the second frailty assessment module applies random forest machine learning of n estimators and a depth d, for example n=20 and d=10.

Advantageously, the inventors tested this second frailty assessment module using the cross-validation technique, and found that it provided an accuracy that ranges from 86% to 91%, with an average accuracy exceeding 89%.

Alternatively, the second frailty assessment module applies other classifiers, such as a multi-layer perceptron (MLP), support vector machines with linear, quadratic or polynomial kernels, a k-Nearest Neighbors (k-NN) classifier, a decision tree with a given depth or a Gradient Boosting Machine (GBM).

Advantageously, the proposed method respects the subject's privacy and is not limited to indoor environments, since it is based on wearable sensors.

Advantageously, the autonomy of the proposed method, device and system is important, since accelerometers and barometers have a low electrical consumption.

Advantageously, the combination of frailty assessment results based on both global features and local features increases accuracy of the estimation and prediction or detection of frailty of the subject.

Advantageously, the proposed method to predict a trend during time, and therefore to predict a transition from robustness to pre-frailty or a transition from pre-frailty to frailty.


**Claims**

1. Method for predicting frailty of a subject, using a wearable device, worn by the subject in unsupervised conditions, the wearable device comprising an accelerometer, the method being implemented by a microcontroller, connected to the accelerometer, the microcontroller being configured to receive acceleration signals from the accelerometer over time, the method being **characterized in that** it comprises the following steps, implemented by the microcontroller:

   a- compute (58) a plurality of global features representative of the acceleration signals captured during a predetermined observation period,
   b- extract (60) segments of acceleration signals within at least one period of walking of the subject within the observation period;
   c- compute (62) a plurality of local features representative of the subject's gait during said at least one period of walking from the magnitude of the segments of acceleration signals,
   d- apply a first frailty assessment module (64) on the plurality of global features to obtain a first output representative of the frailty of the subject,
   e- apply a second frailty assessment module (66) on the plurality of local features to obtain a second output representative of the frailty of the subject,
   f- combine (68) the first output of the first frailty assessment module (64) and the second output of the second frailty assessment module (66) to predict a status of frailty of the subject amongst the classes {robust, pre-frail, frail} for the predetermined observation period.

2. The method according to claim 1, wherein the first output is a first modified vector obtained from a first feature vector of global features and the second output is a second modified vector obtained from a second feature vector of local features, the combining comprising concatenating said first modified vector and said second modified vector into a concatenated vector, the combining (68) comprising providing the concatenated vector to an output layer to obtain the predicted status of frailty of the subject.

3. The method according to claim 1, wherein the first output is a first estimated status of frailty of the subject, the second output is a second estimated status of frailty of the subject, and the combining (68) comprises voting to select the predicted status of frailty of the subject between the first estimated status of frailty of the subject and the second estimated status of frailty of the subject.

4. The method according to any of claims 1 to 3, comprising applying the steps a) to f) over a plurality of successive observation periods to obtain a predicted status of frailty of the subject associated to each observation period of the plurality of successive observation periods, further comprising an analysis step (69) obtaining a trajectory representative of the frailty of the subject over the plurality of successive observation periods, and determining by analysing said trajectory a transition between robust to pre-frail or between pre-frail to frail.

5. The method according to any of claims 1 to 4 comprising a processing (54) of the acceleration signals during the observation period to obtain a list of labels representing a sequence of daily physical activities performed by the subject wearing the wearable device, and further comprising determining (70) active periods and inactive periods based on said list of labels.

6. The method according to claim 5, further comprising computing a plurality of global features (72) relative to the determined active periods during the observation period.

7. The method according to any of claims 1 to 6, further comprising acquiring barometric signals (56) during the observation period and computing (74) at least one altitude related global feature.

8. The method according to any of claims 1 to 7, further comprising computing (76) at least one sleep quality related global feature by analysing acceleration signals captured during a predetermined night period.

9. The method according to any of claims claim 1 to 8, wherein the computing of a plurality of local features comprises computing (86) a normalized auto-correlation signal of the magnitude of the segments of acceleration signals during at least one period of walking, and computing (88) at least one local feature based on said normalized autocorrelation signal.

10. The method according to any of claims 1 to 9, wherein the computing of a plurality of local features further comprises fitting an autoregressive model to the magnitude of the segments of acceleration signals during at least one period of walking, the fitting comprising computing (90) parameters of the autoregressive model, and computing (92) a plurality of local features based on the parameters of the autoregressive model.

11. The method according to any of claims 1 to 10, wherein the first frailty assessment module applies a machine learning classifier on the computed global features.

12. The method according to any of claims 1 to 11, wherein the second frailty assessment module applies a machine learning classifier on the computed local features.

13. Computer program comprising code instructions which, when they are executed by a programmable device, implement a method for predicting frailty of a subject according to claims 1 to 12.

14. Device for predicting frailty of a subject, the device (4) being a wearable device, worn by the subject in unsupervised conditions, comprising an accelerometer (6), and a microcontroller (10) connected to the accelerometer (6), the microcontroller (10) being configured to receive acceleration signals from the accelerometer (6) over time, the device (4) being **characterized in that** the microcontroller (10) is configured to implement:

> • an extracting module (30) configured to extract segments of acceleration signals within at least one period of walking of the subject within an observation period,
> • a global features computing module (32) configured to compute a plurality of global features representative of the acceleration signals captured during a predetermined observation period,
> • a local features computing module (34) configured to compute a plurality of local features representative of the subject's gait during said at least one period of walking from the magnitude of the segments of acceleration signals,
• a first frailty assessment module (36) applied on the plurality of global features to obtain a first output representative of the frailty of the subject,
• a second frailty assessment module (38) applied on the plurality of local features to obtain a second output representative of the frailty of the subject,
• a fusion module (40) configured to combine the first output of the first frailty assessment module and the second output of the second frailty assessment module to predict a status of frailty of the subject amongst the classes {robust, pre-frail, frail} for the predetermined observation period.

FIG.1

FIG.2

EP 4 166 076 A1

```
┌─────────────────────────┐
│                         │
│           70            │
│                         │
└─────────────────────────┘
             │
             ▼
┌─────────────────────────┐
│                         │
│           72            │
│                         │
└─────────────────────────┘
             │
             ▼
┌─────────────────────────┐
│                         │
│           74            │
│                         │
└─────────────────────────┘
             │
             ▼
┌─────────────────────────┐
│                         │
│           76            │
│                         │
└─────────────────────────┘
```

FIG.3

FIG.4

Europäisches
Patentamt
European
Patent Office
Office européen
des brevets

# EUROPEAN SEARCH REPORT

Application Number

**EP 21 30 6449**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2018/117914 A1 (LLC GERO [RU]) 28 June 2018 (2018-06-28) * paragraph [0348] – paragraph [0359] * * figure 5.4 * ----- | 1-14 | INV. A61B5/11 A61B5/00 |
| A | KR 2019 0108016 A (SEOUL NAT UNIV HOSPITAL [KR]) 23 September 2019 (2019-09-23) * abstract * * figures 1-3 * ----- | 1-14 | |
| A | US 2013/110475 A1 (GREENE BARRY R [IE] ET AL) 2 May 2013 (2013-05-02) * the whole document * ----- | 1-14 | |

TECHNICAL FIELDS
SEARCHED      (IPC)

**A61B**

The present search report has been drawn up for all claims

| Place of search | Date of completion of the search | Examiner |
|---|---|---|
| **The Hague** | **10 March 2022** | **Van Dop, Erik** |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

..........................................................................
& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 21 30 6449

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2022

| Patent document cited in search report | | Publication date | Patent family member(s) | | Publication date |
|---|---|---|---|---|---|
| WO 2018117914 | A1 | 28-06-2018 | EP | 3558113 A1 | 30-10-2019 |
| | | | US | 2019365332 A1 | 05-12-2019 |
| | | | WO | 2018117914 A1 | 28-06-2018 |
| KR 20190108016 | A | 23-09-2019 | NONE | | |
| US 2013110475 | A1 | 02-05-2013 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10299736 B2

**Non-patent literature cited in the description**

- **L.P. FRIED et al.** Frailty in older adults: Evidence for a phenotype. *Journal of Gerontology: Medical sciences,* 2001, vol. 56A, 146-156
- **BY M. ABBAS ; R. LE BOUQUIN JEANNES.** Exploiting Local Temporal Characteristics via Multinomial Decomposition Algorithm for Real-Time Activity Recognition. *IEEE Transactions on Instrumentation and Measurement,* September 2021, vol. 70
- **ZHENYU HE ; ZHIBIN LIU ; LIANWEN JIN ; LI-XIN ZHEN ; JIAN-CHENG HUANG.** Weightlessness feature — a novel feature for single tri-axial accelerometer based activity recognition. *2008 19th International Conference on Pattern Recognition,* 2008
- **W. HASKELL et al.** Physical activity and public health: updated recommendation for adults from the American College of Sports Medicine and the American Heart Association. *Medicine & Science in Sports & Exercise,* 2007, vol. 39 (8), 1423-1434